# EUROPÄISCHE PATENTSCHRIFT

(11) **EP 1 369 412 B1**
(45) Veröffentlichungstag und Bekanntmachung des Hinweises auf die Patenterteilung: **27.07.2011**
(21) Anmeldenummer: 03011471.4
(22) Anmeldetag: 21.05.2003
(51) Int. Cl.: C07C 205/45, C07C 205/56, C07C 211/60, C07C 209/22, C07C 209/36, C07C 263/10

(54) **Verfahren zur Herstellung von 5-Nitro-3,4-dihydro-1(2H)-naphthalinon, 1,5-Naphtalindiamin und 1,5-Naphtalindiisocyanat**
Process for the preparation of 5-nitro-3,4-dihydro-1(2H)-naphthalenone, 1,5-naphthalendiamine and 1,5-naphthalendiisocyanate
Procédé pour la préparation de 5-nitro-3,4-dihydro-1(2H)-naphthalénone, 1,5-naphthalènediamine et 1,5-naphthalènediisocyanate

(30) Priorität: 03.06.2002 DE 10224463
(43) Veröffentlichungstag der Anmeldung: 10.12.2003
(73) Patentinhaber: Bayer MaterialScience AG, 51368 Leverkusen (DE)
(72) Erfinder: Zechlin, Joachim, Dr., 41472 Neuss (DE); Verkerk, Kai, Dr., 40723 Hilden (DE); Wastian, Dietmar, 41542 Dormagen (DE); Joschek, Katrin, Dr., 51061 Köln (DE); Loddenkemper, Tim, 41542 Dormagen (DE); Pinke, Wilfried, 41542 Dormagen (DE); Schelhaas, Michael, Dr., 50733 Köln (DE); Ronge, Georg, Dr., 40549 Düsseldorf (DE)

(56) Entgegenhaltungen:
- EP-A- 1 295 864
- WO-A-02/051792
- DE-A- 19 651 041
- DE-C- 397 150
- GB-A- 1 543 276
- US-A- 3 448 139

## Beschreibung

Die Erfindung betrifft ein Verfahren zur Herstellung von 5-Nitro-3,4-dihydro-1(2H)-naphthalinon durch Hydrolyse von 4-(2-Nitrophenyl)-n-butyronitril und durch Umsetzung der entstandenen 4-(2-Nitrophenyl)-n-buttersäure.

Das Dokument DE-A-397 150 offenbart die Herstellung von 5-Nitro-3,4-dihydro-1(24)-Naphthalinon durch Oxidation von 1-Nitronaphthalen.

5-Nitro-3,4-dihydro-1(2H)-naphthalinon ist ein Intermediat für die Herstellung von 1,5-Naphthalindiamin, das durch Aminierung, Aromatisierung und anschließende Hydrierung aus 5-Nitro-3,4-dihydro-1(2H)-naphthalinon erhalten werden kann. 1,5-Naphthalindiamin kann anschließend durch Phosgenierung zu 1,5-Naphthalindiisocyanat weiter umgesetzt werden.

Verschiedene Verfahren zur Herstellung von 1,5-Naphthalindiamin sind in der Literatur bereits bekannt. Im Allgemeinen geht die Darstellung von 1,5-Naphthalindiamin von Naphthalin aus, das geeignet substituiert wird. So wird in JP-A2-07 278 066 die Synthese von 1,5-Naphthalindiamin über einen Amin-Brom-Austausch an 1,5-Bromaminonaphthalin beschrieben. Das benötigte Edukt wird bei diesem Verfahren durch Bromierung von 1-Nitronaphthalin erzeugt.

In JP-A2-04 154 745, JP-A2-56 059 738 und DE-A1-2 523 351 wird die Synthese von 1,5-Naphthalindiamin in Kombination mit 1,8-Naphthalindiamin durch Reduktion eines Gemisches aus 1,5- und 1,8-Dinitronaphthalin beschrieben. In DE-C1-3 840 618 wird die Synthese von 1,5-Naphthalindiamin durch alkalische Hydrolyse von Dinatrium Naphthalin-1,5-disulfonat und anschließende Umsetzung mit Ammoniak beschrieben.

Alle diese Verfahren haben den Nachteil, dass das Produkt oder ein im Laufe des Verfahrens erzeugtes Zwischenprodukt als Isomerenmischung anfällt, die neben dem 1,5-Isomeren noch weitere Isomere enthält, die abgetrennt werden müssen. Zudem verläuft insbesondere das in DE-C1-3 840 618 beschriebene Verfahren unter sehr drastischen und korrosiven Reaktionsbedingungen ab.

Aufgabe der vorliegenden Erfindung ist daher, ein einfaches Verfahren zur Herstellung von Intermediaten für die Produktion von 1,5-Naphthalindiamin bereit zu stellen, nach dem 1,5-Naphthalindiamin hergestellt werden kann, ohne dass andere Isomere in nennenswerten Mengen anfallen und abgetrennt werden müssen. Aufgabe der vorliegenden Erfindung ist weiterhin die Bereitstellung von Verfahren zur Herstellung von 1,5-Naphthalindiamin und 1 ,5-Naphthalindiisocyanat auf Basis dieser Intermediate.

Es wurde nun ein Verfahren gefunden, durch das ausgehend von 4-(2-Nitrophenyl)-n-butyronitril 5-Nitro-3,4-dihydro-1(2H)-naphthalinon als Intermediat für die Herstellung von 1,5-Naphthalindiamin einfach und weitgehend isomerenrein dargestellt werden kann.

Die Erfindung betrifft ein Verfahren zur Herstellung von 5-Nitro-3,4-dihydro-1(2H)-naphthalinon, das einen Schritt enthält, in dem man 4-(2-Nitrophenyl)-n-butyronitril zu 4-(2-Nitrophenyl)-n-buttersäure umsetzt.

4-(2-Nitrophenyl)-n-butyronitril kann aus ortho-Nitrotoluol und Acrylnitril bevorzugt bei Temperaturen von -10°C bis 100°C hergestellt werden. Besonders bevorzugt arbeitet man bei 20°C bis 75°C, ganz besonders bevorzugt bei Temperaturen von 30°C bis 60°C.

Die Reaktion wird basenkatalysiert durchgeführt. Als Basen können Oxide, Hydroxide und Carbonate von Lithium, Natrium, Kalium, Rubidium, Cäsium, Magnesium, Calcium, Strontium, Barium oder Aluminium sowie Mischungen daraus eingesetzt werden. Besonders geeignet sind Natrium- und Kaliumhydroxid. Bevorzugt werden Lösungen der Base eingesetzt. Die Lösungen der Base können auch in Kombination mit einem Phasentransferkatalysator eingesetzt werden. Solche Phasentransferkatalysatoren sind z.B. quartäre Ammoniumsalze. Geeignete Ammoniumverbindungen sind Tetraalkylammonium-Halogenide und -Hydrogensulfate wie Tributylmethylammonium-Chlorid, Trioctylammonium-Chlorid, TetrabutylammoniumChlorid oder Tetrabutylammonium-Hydrogensulfat. Ebenso geeignet ist die Verwendung von entsprechenden Tetraalkyl- bzw. Tetraaryl-Phosphoniumsalzen wie Tetramethylphosphonium-Bromid und Tetraphenylphosphonium-Bromid sowie die Verwendung von Löslichkeitsvermittlem wie Polyethylenglycoldimethylethem.

Bevorzugt werden die Lösungen der Base ohne Phasentransferkatalysator eingesetzt.

Als Lösungsmittel sind grundsätzlich Wasser sowie alle basenstabilen organischen Lösungsmittel geeignet. Bevorzugt werden aromatische Lösungsmittel wie Benzol, Toluol, Xylol, Monochlorbenzol, Dichlorbenzol, Trichlorbenzol, Nitrobenzol oder Nitrotoluol sowie Dimethylsulfoxid, Dimethylformamid und aliphatische Kohlenwasserstoffe wie Ligroin, Cyclohexan, Pentan, Hexan, Heptan, Octan eingesetzt. Bevorzugt wird Dimethylsulfoxid in einer Konzentration von 10-80 Gew.-% bezogen auf ortho-Nitrotoluol eingesetzt, besonders bevorzugt in einer Konzentration von 30 - 60 Gew.-%.

Ortho-Nitrotoluol wird vorzugsweise im Überschuss eingesetzt. Bevorzugt werden pro mol Acrylnitril 1 bis 40 mol ortho-Nitrotoluol eingesetzt, besonders bevorzugt 5 bis 20 mol ortho-Nitrotoluol.

In einer ersten bevorzugten Ausführungsform enthält das Verfahren zur Herstellung von 5-Nitro-3,4-dihydro-1(2H)-naphthalinon die Schritte:
a) Umsetzung von 4-(2-Nitrophenyl)-n-butyronitril mit einer Säure oder einer Base zu 4-(2-Nitrophenyl)-n-buttersäure,
b) Cyclisierung der in Schritt a) gebildeten 4-(2-Nitrophenyl)-n-buttersäure zu 5-Nitro-3,4-dihydro-1 (2H)-naphthalinon

Die Hydrolyse von 4-(2-Nitrophenyl)-n-butyronitril zu 4-(2-Nitrophenyl)-n-buttersäure wird in Substanz oder in einem Lösemittel in Gegenwart einer oder mehrerer Säuren oder einer oder mehrerer Basen durchgerührt.

Geeignete Säuren sind starke Säuren wie verdünnte oder konzentrierte Mineralsäuren wie Salzsäure, Schwefelsäure, Phosphorsäure sowie Hydrogensulfate und Dihydrogenphosphate.

Geeignete Basen sind starke Basen wie Oxide, Hydroxide und Carbonate von Lithium, Natrium, Kalium, Rubidium, Cäsium, Magnesium, Calcium, Strontium, Barium oder Aluminium sowie Mischungen daraus sowie wässrige Lösungen oder Suspensionen von Hydroxiden und Carbonaten von Lithium, Natrium, Kalium, Rubidium, Cäsium, Magnesium, Calcium, Strontium, Barium oder Aluminium. Besonders geeignet sind wässrige Lösungen von Natrium- oder Kaliumhydroxid.

Geeignete Lösmittel sind lineare, verzweigte oder cyclische aliphatische Kohlenwasserstoffe wie Ligroin oder Cyclohexan, Pentan, Hexan, Heptan, Octan sowie aromatische Lösungsmittel wie Benzol, Toluol, Xylol, Monochlorbenzol, Dichlorbenzol, Trichlorbenzol. Bevorzugt wird in Substanz gearbeitet.

Pro mol 4-(2-Nitrophenyl)-n-butyronitril werden bevorzugt 0,5 bis 20 mol Säure oder Base, besonders bevorzugt 1 bis 10 mol Säure oder Base, ganz besonders bevorzugt 1,5 bis 6 mol Säure oder Base eingesetzt.

Die Reaktion wird bevorzugt bei Temperaturen von 0°C bis 150°C durchgerührt, besonders bevorzugt zwischen 30°C bis 120°C, ganz besonders bevorzugt zwischen 500°C und 100°C.

Die Cyclisierung von 4-(2-Nitrophenyl)-n-buttersäure zu Nitro-3,4-dihydro-1(2H)-naphthalinon wird in Substanz oder in einem Lösemittel in Gegenwart einer oder mehrerer Säuren durchgeführt. Geeignete Säuren sind starke Lewis- oder Bronstedsäuren wie z.B. Aluminiumtrichlorid, Bortrifluorid, Schwefelsäure, Phosphorsäure, Polyphosphorsäure, Phosphorpentoxid, Methansulfonsäure, Trifluormethansulfonsäure, Trifluoressigsäure, Niobsäure oder Gemische aus Antimonpentafluorid und Fluorschwefelsäure. Es können auch Mischungen der Säuren eingesetzt werden. Es können auch heterogenisierte Säuren wie z.B. Polyphosphorsäure oder teil- oder perfluorierte Aryl- oder Alkylsulfonsäuren auf Trägermaterialien wie Siliciumdioxid, Dialuminiumtrioxid oder auch teil- oder perfluorierte polymere Aryl- oder Alkylsulfonsäuren eingesetzt werden.

Pro mol 4-(2-Nitrophenyl)-n-buttersäure werden bevorzugt 0,1 bis 100 mol Säure, besonders bevorzugt 0,5 bis 50 mol Säure, ganz besonders bevorzugt 1 bis 25 mol Säure eingesetzt.

Die Reaktion wird bevorzugt bei Temperaturen von 50°C bis 300°C durchgeführt, besonders bevorzugt zwischen 100°C bis 250°C, ganz besonders bevorzugt zwischen 120°C und 230°C.

Geeignete Lösmittel sind lineare, verzweigte oder cyclische aliphatische Kohlenwasserstoffe wie Ligroin oder Cyclohexan, Pentan, Hexan, Heptan, Octan, aromatische Lösemittel wie Benzol, Toluol, Xylol, Monochlorbenzol, Dichlorbenzol, Trichlorbenzol, Lösemittel wie Dimethylsulfoxid, Dimethylformamid sowie hochtemperaturfeste, hochsiedende Alkylbenzol- oder Benzolderivate wie isomere Dibenzyltoluole, Di- und Triarylalkyle, Bi- oder Triaryloxide, Terphenyle und ihre teilhydrierten und hydrierten Analoga, alkylierte oder nichtalkylsubstituierte Benzyltoluole. Es kann aber auch in Substanz gearbeitet werden.

Alle Reaktionsschritte können kontinuierlich oder diskontinuierlich, beispielsweise in Rührkesselreaktoren oder Rohrreaktoren, durchgeführt werden.

In einer zweiten bevorzugten Ausführungsform enthält das Verfahren zur Herstellung von 5-Nitro-3,4-dihydro-1(2H)-naphthalinon die Schritte:
a) Umsetzung von 4-(2-Nitrophenyl)-n-butyronitril mit einer Säure oder einer Base zu 4-(2-Nitrophenyl)-n-buttersäure,
b) Chlorierung der in Schritt a) gebildeten 4-(2-Nitrophenyl)-n-buttersäure zum 4-(2-Nitrophenyl)-n-buttersäurechlorid,
c) Cyclisieren des in Schritt b) gebildeten 4-(2-Nitrophenyl)-n-buttersäurechlorids zum 5-Nitro-3,4-dihydro-1(2H)-naphthalinon

Die Umsetzung (Hydrolyse) von 4-(2-Nitrophenyl)-n-butyronitril mit einer Säure oder einer Base zu 4-(2-Nitrophenyl)-n-buttersäure wird analog zu Schritt a) der ersten bevorzugten Ausführungsform in Substanz oder in einem Lösemittel in Gegenwart einer oder mehrerer Säuren oder einer oder mehrerer Basen durchgeführt.

Geeignete Säuren sind starke Säuren wie verdünnte oder konzentrierte Mineralsäuren wie Salzsäure, Schwefelsäure, Phosphorsäure sowie Hydrogensulfate und Dihydrogenphosphate.

Geeignete Basen sind starke Basen wie Oxide, Hydroxide und Carbonate von Lithium, Natrium, Kalium, Rubidium, Cäsium, Magnesium, Calcium, Strontium, Barium oder Aluminium sowie Mischungen daraus sowie wässrige Lösungen oder Suspensionen von Hydroxiden und Carbonaten von Lithium, Natrium, Kalium, Rubidium, Cäsium, Magnesium, Calcium, Strontium, Barium oder Aluminium. Besonders geeignet sind wässrige Lösungen von Natrium- oder Kaliumhydroxid.

Geeignete Lösmittel sind lineare, verzweigte oder cyclische aliphatische Kohlenwasserstoffe wie Ligroin oder Cyclohexan, Pentan, Hexan, Heptan, Octan sowie aromatische Lösungsmittel wie Benzol, Toluol, Xylol, Monochlorbenzol, Dichlorbenzol, Trichlorbenzol. Bevorzugt wird in Substanz gearbeitet.

Pro mol 4-(2-Nitrophenyl)-n-butyronitril werden bevorzugt 0,5 bis 20 mol Säure oder Base, besonders bevorzugt 1 bis 10 mol Säure oder Base, ganz besonders bevorzugt 1,5 bis 6 mol Säure oder Base eingesetzt.

Die Reaktion wird bevorzugt bei Temperaturen von 0°C bis 150°C durchgeführt, besonders bevorzugt zwischen 30°C bis 120°C, ganz besonders bevorzugt zwischen 50°C und 100°C.

Die Chlorierung der 4-(2-Nitrophenyl)-n-buttersäure zum 4-(2-Nitrophenyl)-n-buttersäurechlorid wird mit Chlorierungsmitteln wie Chlor, Thionylchlorid, Phosgen, Phosphorylchlorid, Phosphortrichlorid oder Phosphorpentachlorid durchgeführt. Bevorzugt wird Thionylchlorid oder Phosgen eingesetzt.

Geeignete Lösungsmittel sind lineare, verzweigte oder cyclische aliphatische Kohlenwasserstoffe wie Ligroin oder Cyclohexan, Pentan, Hexan, Heptan, Octan sowie aromatische Lösungsmittel wie Benzol, Xylol, Monochlorbenzol, Dichlorbenzol, Trichlorbenzol. Bevorzugt wird in Substanz gearbeitet.

Die Reaktion wird bevorzugt bei Temperaturen von 0°C bis 250°C durchgeführt, besonders bevorzugt zwischen 20°C bis 200°C, ganz besonders bevorzugt zwischen 30°C und 180°C.

Die Cyclisierung des in Schritt b) gebildeten 4-(2-Nitrophenyl)-n-buttersäurechlorids zum 5-Nitro-3,4-dihydro-1(2H)-naphthalinon wird in Substanz oder in einem Lösemittel in Gegenwart von Säuren durchgeführt. Geeignete Säuren sind starke Lewisoder Bronstedsäuren wie z.B. Aluminiumtrichlorid, Eisentrichlorid, Zinndichlorid, Titantetrachlorid, Bortrifluorid, Schwefelsäure, Phosphorsäure, Polyphosphorsäure, Phosphorpentoxid, Methansulfonsäure, Trifluormethansulfonsäure, Trifluoressigsäure, Niobsäure oder Gemische aus Antimonpentafluorid und Fluorschwefelsäure. Es können auch Mischungen der Säuren eingesetzt werden. Es können auch heterogenisierte Säuren wie z.B. Polyphosphorsäure oder teil- oder perfluorierte Aryl- oder Alkylsulfonsäuren auf Trägermaterialien wie Siliciumdioxid, Dialuminiumtrioxid oder auch teil- oder perfluorierte polymere Aryl- oder Alkylsulfonsäuren eingesetzt werden.

Geeignete Lösemittel sind lineare, verzweigte oder cyclische aliphatische Kohlenwasserstoffe wie Ligroin oder Cyclohexan, Pentan, Hexan, Heptan, Octan, aromatische Lösemittel wie Monochlorbenzol, Dichlorbenzol, Trichlorbenzol, Lösemittel wie Dimethylsulfoxid, Dimethylformamid sowie hochtemperaturfeste, hochsiedende Alkylbenzol- oder Benzolderivate wie isomere Dibenzyltoluole, Di- und Triarylalkyle, Bi- oder Triaryloxide, Terphenyle und ihre teilhydrierten und hydrierten Analoga, alkylierte oder nichtalkylsubstituierte Benzyltoluole. Es kann aber auch in Substanz gearbeitet werden.

Pro mol 4-(2-Nitrophenyl)-n-buttersäurechlorid werden bevorzugt 0,1 bis 100 mol Säure, besonders bevorzugt 0,5 bis 50 mol Säure, ganz besonders bevorzugt 1 bis 25 mol Säure eingesetzt.

Die Reaktion wird bevorzugt bei Temperaturen von 50°C bis 300°C durchgeführt, besonders bevorzugt zwischen 100°C bis 250°C, ganz besonders bevorzugt zwischen 120°C und 230°C.

Alle Reaktionsschritte können kontinuierlich oder diskontinuierlich, beispielsweise in Rührkesselreaktoren oder Rohrreaktoren, durchgeführt werden.

Die Erfindung betrifft auch ein Verfahren zur Herstellung von 1,5-Naphthalindiamin, das einen Schritt enthält, in dem man 4-(2-Nitrophenyl)-n-butyronitril zu 4-(2-Nitrophenyl)-n-buttersäure umsetzt.

Ausgehend von 5-Nitro-3,4-dihydro-1(2H)-naphthalinon wird 1,5-Naphthalindiamin durch Aminierung, Aromatisierung und Hydrierung erhalten.

Die Aminierung von 5-Nitro-3,4-dihydro-1(2H)-naphthalinon zum Nitro-Imin bzw. Nitro-Enamin geschieht durch Umsetzung mit Ammoniak, vorzugsweise in Gegenwart von Ammoniumsalzen wie Ammoniumchlorid.

Die Aromatisierung bzw. Dehydrierung des Nitro-Enamins 5-Nitro-3,4-dihydro-1-naphthylamin bzw. des Nitro-Imins 5-Nitro-3,4-dihydro-1(2H)-naphthylimin zu 5-Nitro-1-naphthylamin oder 5-Nitroso-1-naphthylamin oder einem Gemisch der Verbindungen wird, beispielsweise in einem inerten Lösungsmittel ohne Katalysator oder in Gegenwart eines Katalysators durchgeführt. Dabei kann neben dem dehydrierten Produkt 5-Nitro- 1 -naphthylamin auch formal durch Synproportionierung 5-Nitroso-1-naphthylamin entstehen. In Spuren entsteht auch 1,5-Naphthalindiamin. Die Produkte können in beliebigen Mischungsverhältnissen weiter verarbeitet werden.

Geeignete Lösungsmittel sind Ammoniak und lineare, verzweigte oder cyclische aliphatische Kohlenwasserstoffe wie Ligroin oder Cyclohexan, sowie Acetonitril, Dimethylformamid, Dimethylacetamid und aromatische Lösungsmittel wie Benzol, Toluol, Xylol, Nitrobenzol, Nitrotoluol oder Monochlorbenzol, Dichlorbenzol, Trichlorbenzol. Die Aromatisierung kann auch in Abwesenheit eines Lösungsmittels durchgeführt werden.

Geeignete Katalysatoren sind Dehydrierungskatalysatoren, die in der Literatur beschrieben sind (Römpp Lexikon Chemie; Georg Thieme Verlag, Stuttgart, 10. Auflage 1997, S. 891, Kapitel "Dehydrierung", 1. Abschnitt; Ullmann's Encyclopedia of Industrial Chemistry, VCH Verlagsgesellschaft mbH, Weinheim, 5. Auflage 1989, Vol A13, Kapitel "Hydrogenation and Dehydrogenation", Unterkapitel 2. "Dehydrogenation", S. 494-497). Dazu gehören die Metalle der 8.-10. Gruppe des Periodensystems (G. J. Leigh [Editor], Nomenclature of Inorganic Chemistry, Recommendations 1990, Blackwell Scientific Publications, Oxford, Chapter 1-3.8.1 "Groups of Elements in the Periodic Table and their Subdivision, p. 41-43.), insbesondere Platin, Palladium, Ruthenium und Iridium, Eisen, Cobalt, Nickel und Kombinationen davon.

Die Metalle können auch gemeinsam mit weiteren Metallen wie Lanthan, Scandium, Vanadium, Chrom, Molybdän, Wolfram, Mangan, Zinn, Zink, Kupfer, Silber oder Indium eingesetzt werden. Die genannten Metalle können dabei als reine Elemente, als Oxide, Sulfide, Halogenide, Carbide oder Nitride vorliegen oder aber kombiniert mit organischen Liganden eingesetzt werden. Als Liganden geeignet sind Kohlenwasserstofiverbindungen mit Donorgruppen wie beispielsweise Amine, Nitrile, Phosphine, Thiole, Thioether, Alkohole, Ether oder Carbonsäuren. Gegebenenfalls sind die Katalysatoren auf einem Trägermaterial aufgebracht. Geeignete Trägermaterialien sind Aktivkohle, Aluminiumoxid, Siliciumoxid, Zirkonoxid, Zinkoxid, Zeolithe.

Gegebenenfalls wird in Gegenwart eines Oxidationsmittels wie Sauerstoff oder Luft gearbeitet. Die Reaktion wird im Allgemeinen bei Temperaturen von 50°C bis 250°C durchgeführt, bevorzugt bei 100°C bis 200°C.

Die anschließende Hydrierung von 5-Nitro-1-naphthylamin oder 5-Nitroso-1-naphthylamin oder einem Gemisch der Verbindungen zu 1,5-Naphthalindiamin wird in Anwesenheit eines Hydrierkatalysators durchgeführt.

Als Hydrierkatalysatoren eignen sich praktisch alle heterogenen Katalysatoren, die als Hydrierkatalysatoren bekannt sind (Römpp Lexikon Chemie; Georg Thieme Verlag, Stuttgart, 10. Auflage 1997, S. 1831, Kapitel "Hydrierung"; Ullmann's Encyclopedia of Industrial Chemistry, VCH Verlagsgesellschaft mbH, Weinheim, 5. Auflage 1989, Vol A13, Kapitel "Hydrogenation and Dehydrogenation", Unterkapitel 1.2 "Catalysts", S. 488). Bevorzugte Katalysatoren sind die Metalle der 8.-10. Gruppe des Periodensystems (G. J. Leigh [Editor], Nomenclature of Inorganic Chemistry, Recommendations 1990, Blackwell Scientific Publications, Oxford, Chapter I-3.8.1 "Groups of Elements in the Periodic Table and their Subdivision, p. 41-43.), Kupfer oder Chrom auf geeignetem Träger mit einem Metallgehalt von 0,01 bis 50 Gew.-%, bevorzugt 0,1 bis 20 Gew.-%, bezogen auf das Gesamtgewicht des Katalysators. Es können auch Katalysatoren eingesetzt werden, die eines oder mehrere der oben genannten Metalle enthalten. Bevorzugte Metalle sind insbesondere Platin, Palladium und Rhodium, besonders bevorzugt sind Platin und Palladium. Weitere bevorzugte Katalysatoren sind Raney-Nickel und geträgerte Nickelkatalysatoren. Es können auch die obengenannten Metalle oder ihre Verbindungen in reiner Form als Feststoff eingesetzt werden. Als Beispiele für ein Metall in reiner Form seien Palladium- und Platinschwarz genannt.

Die Katalysatoren können in diskontinuierlichen Verfahrensvarianten in Mengen von 0,01 bis 50 Gew.-%, bezogen auf eingesetztes 5-Nitro- bzw. 5-Nitroso-1-naphthylamin verwendet werden, bevorzugt in Mengen von 0,01 bis 20 Gew.-%, besonders bevorzugt in Mengen von 0,01 bis 10 Gew.-%. Bei kontinuierlicher Durchführung der Reaktion, beispielsweise in einem Rührkessel mit pulverförmigen Katalysator oder in der Rieselphase am Festbettkatalysator, können Belastungen von 0,01 bis 500 g, bevorzugt 0,1 - 200 g, besonders bevorzugt 1 bis 100 g 5-Nitro- bzw. 5-Nitroso-1-naphthylamin pro g Katalysator und Stunde eingestellt werden.

Die Reaktionstemperaturen betragen im Allgemeinen - 20°C bis 150°C, insbesondere 40°C bis 120°C; der Wasserstoffdruck liegt im Allgemeinen bei 0,1 bis 150 bar, insbesondere bei 0,5 bis 70 bar, ganz besonders bevorzugt bei 1 bis 50 bar.

Bevorzugt wird für die Aromatisierung (Dehydrierung) und die anschließende Hydrierung der gleiche Katalysator verwendet, wobei die beiden Schritte nacheinander in einem Reaktionsgefäß durchgeführt werden können.

Geeignete Lösungsmittel für die Hydrierung sind lineare oder verzweigte aliphatische Alkohole wie Methanol, Ethanol, Propanol, Butanol, lineare, verzweigte oder cyclische aliphatische Kohlenwasserstoffe wie Ligroin oder Cyclohexan, sowie Dimethylformamid, Dioxan, Dimethylacetamid und aromatische Lösungsmittel wie Benzol, Toluol, Xylol, Nitrobenzol, Nitrotoluol oder Monochlorbenzol, Dichlorbenzol, Trichlorbenzol.

Das 1,5-Naphthalindiamin kann in an sich bekannter Weise zum 1,5-Naphthalindiisocyanat phosgeniert werden.

### Beispiele

### Erste bevorzugte Ausführungsform:

### Beispiel 1: Darstellung von 4-(2-Nitrophenyl)-n-buttersäure durch Hydrolyse von 4-(2-Nitrophenyl)-n-butyronitril mit Phosphorsäure

190 g 4-(2-Nitrophenyl)-n-butyronitril (1 mol) und 1153 g Phosphorsäure (85 Gew.-%) (10 mol) werden in einer 2 L Vierhalskolbenrührapparatur mit Blattrührer, Thermometer und Kühler vorgelegt. Unter Rühren wird auf 100°C erhitzt und 17 Stunden bei 100°C gerührt. Anschließend wird unter Rühren langsam auf Raumtemperatur abgekühlt, wobei die emulgierte 4-(2-Nitrophenyl)-n-buttersäure erstarrt. Die überschüssige Phosphorsäure wird dekantiert und der Feststoff in 1000 ml Chloroform gelöst. Die Lösung wird in einen Scheidetrichter überführt und dreimal mit je 50 ml destilliertem Wasser Phosphorsäure-frei gewaschen und in einem Rotationsverdampfer bei 80°C und 20 mbar eingeengt. Der Rückstand (4-(2-Nitrophenyl)-nbuttersäure) wird zerstoßen und in einem Exsikkator über di-Phosphorpentoxid bis zur Massenkonstanz getrocknet.

Die dekantierte Phosphorsäure wurde mit 2 L destilliertem Wasser verdünnt und mit 500 ml Chloroform extrahiert. Der Chloroformextrakt wird in einem Rotationsverdampfer bei 80°C und 20 mbar eingeengt und der Rückstand (4-(2-Nitrophenyl)-nbuttersäure) mit dem Rückstand der Trocknung vereinigt.

Auswaage an (4-(2-Nitrophenyl)-n-buttersäure): 200,2 g entsprechend 95,8 % Rohausbeute (Reinheit: 99,3 % ermittelt durch Gaschromatograph (GC)).

### Beispiel 2: Darstellung von 5-Nitro-3,4-dihydro-1(2H)-naphthalinon durch Cyclisierung von 4-(2-Nitrophenyl)-n-buttersäure mit Polyphosphorsäure

In einem Zweihalskolben mit Magnetrührer, Kolonnenkopf, Vakuumvorstoß, Vorlagekolben und beheizbaren Dosiertrichter werden 50 g Dibenzyltoluol (Isomerengemisch, Handelsname Marlotherm SH, Sasol Germany GmbH) und 10 g Polyphosphorsäure (85 Gew.-% P₄O₁₀) vorgelegt. Der auf 90°C beheizte Dosiertrichter wird mit einer Lösung von 2 g 4-(2-Nitrophenyl)-n-buttersäure (NPBS, 9,57 mmol) in 20 g Dibenzyltoluol (Isomerengemisch, Handelsname Marlotherm SH, Sasol Germany GmbH) beschickt. Die Apparatur wird evakuiert und unter Rühren auf Rückfluss erhitzt (163°C, 0,2 mbar). Anschließend tropft man die Lösung kontinuierlich innerhalb von 20 Minuten zu. Die Destillatabnahme wird entsprechend dem Zulauf eingestellt, so dass das Reaktionsvolumen im Sumpfkolben konstant bleibt. Nach vollständiger Zugabe der Lösung wird mit 20 g Dibenzyltoluol (Isomerengemisch, Handelsname Marlotherm SH, Sasol Germany GmbH) nachgespült und sauber destilliert.

Das Destillat enthält 626 mg 5-Nitro-3,4-dihydro-1(2H)-naphthalinon (NT) und 1268 mg NPBS (ermittelt durch GC/ Interne Standard-Methode):

| | |
|---|---|
| Ausbeute NT | 34,2 % |
| Umsatz NPBS | 36,8 % |
| Selektivität | 93,1 % |

### Beispiel 3: Darstellung von 5-Nitro-3,4-dihydro-1(2H)-naphthalinon durch Cyclisierung von 4-(2-Nitrophenyl)-n-buttersäure mit Trifluormethansulfonsäure

In einem 100 ml Zweihalskolben mit Rückflusskühler und Magnetrührer werden 1 g 4-(2-Nitrophenyl)-n-buttersäure (NPBS, 4,78 mmol) und 18 g Trifluormethansulfonsäure (TFMS) bei 20°C vorgelegt (119,9 mmol). Die Lösung wird unter Rühren mit einem auf 130°C vorgeheizten Ölbad aufgeheizt und 45 Minuten bei 130°C gerührt. Die Lösung wird abgekühlt und in eine Mikrodestillationsapparatur überführt. Bei einer Kopftemperatur von 52°C und 0,5 mbar wird der Großteil der überschüssigen TFMS abdestilliert. Der Sumpf enthält 0,914 g 5-Nitro-3,4-dihydro-1(2H)-naphthalinon und 3,44 g TFMS (ermittelt durch GC-ISTD/ Interne Standard-Methode):

| | |
|---|---|
| Ausbeute NT | 99,3 % |
| Umsatz NPBS | 100 % |
| Selektivität | 99,3 % |

### Zweite bevorzugte Ausführungsform:

### Beispiel 4: Darstellung von 4-(2-Nitrophenyl)-n-huttersäurechlorid durch Chlorierung von 4-(2-Nitrophenyl)-n-buttersäure mit Thionylchlorid

In einer 4 L Vierhalskolbenrührapparatur mit Blattrührer, Thermometer, Kühler und Trockenrohr werden 744,3 g 4-(2-Nitrophenyl)-n-buttersäure (NPBS, 3,56 mol) vorgelegt. Unter Rühren werden bei Raumtemperatur 635,5 g Thionychlorid (5,34 mol) zugetropft. Dabei fällt die Temperatur bis auf -6°C. Mit einem Heizpilz wird langsam bis auf Rückfluss (max. 80°C) erwärmt. Dabei entsteht unter starker Gasentwicklung eine rotbraune, leicht viskose Flüssigkeit. Es wird eine Stunde unter Rückflussbedingungen nachgerührt. Anschließend wird das überschüssige Thionylchlorid unter Wasserstrahlvakuum bei 80°C abdestilliert. Das Säurechlorid wird durch Flashdestillation in einer Heißluftdestillationstemperatur (260°C Heißluft, Sumpftemperatur < 180°C, Druck < 1 mbar) gereinigt.

Destillat: 767,7 g 4-(2-Nitrophenyl)-n-buttersäurechlorid entsprechend 94,8 % Rohausbeute (Reinheit: 92,7 % ermittelt durch GC).

### Beispiel 5: Darstellung von 4-(2-Nitrophenyl)-n-buttersäurechlorid durch Chlorierung von 4-(2-Nitrophenyl)-n-buttersäure mit Phosgen

In einem 250 ml Vierhalskolben mit Rührer, Thermometer, Kühler (mit Anschluss an einen Phosgenvernichterturm) werden bei 35°C 20 g 4-(2-Nitrophenyl)-n-buttersäure (95,7 mmol) in 180 g 1,2,4-Trichlorbenzol gelöst. Über ein getauchtes Glasrohr werden 100 g/h Phosgen eingeleitet. Unter Rühren wird innerhalb von 20 Minuten auf 180°C aufgeheizt und 2 Stunden mit 100 g/h Phosgen chloriert. Die tief orange-braun gefärbte Lösung wird bei 100°C durch Strippen mit Stickstoff entphosgeniert. In einer Heißluftdestillationsapparatur (140°C Heißlufttemperatur, Druck <1 mbar) wird das 1,2,4-Trichlorbenzol abdestilliert.

Rückstand: 21,3 g 4-(2-Nitrophenyl)-n-buttersäurechlorid entsprechend 97,7 % Rohausbeute (Reinheit: 91,0 % ermittelt durch GC).

### Beispiel 6: Darstellung von 5-Nitro-3,4-dihydro-1(2H)-naphthalinon durch Cyclisierung von 4-(2-Nitrophenyl)-n-buttersäurechlorid mit Aluminiumtrichlorid

In einer 10 L Vierhalskolbenrührapparatur mit Blattrührer, Thermometer, Kühler, Trockenrohr und Pulverdosiertrichter werden 762,2 g 4-(2-Nitrophenyl)-n-buttersäurechlorid (aus Beispiel 4) in 4,5 L Schwefelkohlenstoff gelöst. Bei 15°C werden 535,8 g Aluminiumchlorid (4,0 mol) über einen Pulverdosiertrichter zugegeben. Dabei geht das Aluminiumchlorid fast vollständig in Lösung und die Sumpftemperatur steigt auf 30°C. Nach kurzer Zeit bilden sich zwei Phasen, die untere Keton-Aluminiumchloridkomplex-Phase ist rotbraun und ölig. Nach vollständiger Aluminiumchloridzugabe rührt man noch 4 Stunden bei Raumtemperatur nach, dabei erstarrt die ölige Phase vollständig. Anschließend wird der Schwefelkohlenstoff dekantiert und zur Zersetzung des Keton-Aluminiumchloridkomplexes wird die feste Phase unter Kühlung mit 2,6 L destilliertem Wasser versetzt. Die Zugabe von 2,6 L Dichlormethan beschleunigt die Hydrolyse und löst das gebildete 5-Nitro-3,4-dihydro-1(2H)-naphthalinon vollständig. Die organische Phase wird dreimal mit insgesamt 1,4 L destilliertem Wasser, einmal mit 1000 ml Natronlauge (1 Gew.-% NaOH) und anschließend mit destilliertem Wasser neutral gewaschen. Mit einem Rotationsverdampfer wird die Lösung eingeengt und das ausgefallene 5-Nitro-3,4-dihydro-1(2H)-naphthalinon wird im Exsikkator über di-Phosphorpentoxid bis zur Massenkonstanz getrocknet.

Auswaage: 548,5 g 5-Nitro-3,4-dihydro-1(2H)-naphthalinon entsprechend 85,7 % Rohausbeute (Reinheit: 99,5 % ermittelt durch GC).

### Beispiel 7: Darstellung von 5-Nitro-3,4-dihydro-1(2H)-naphthalinon durch Cyclisierung von 4-(2-Nitrophenyl)-n-buttersäurechlorid mit Polyphosphorsäure

In einem Zweihalskolben mit Magnetrührer, Kolonnenkopf, Vakuumvorstoß, Vorlagekolben und beheizbaren Dosiertrichter werden 50 g Dibenzyltoluol (Isomerengemisch, Handelsname Marlotherm SH, Sasol Germany GmbH) und 10 g Polyphosphorsäure (85 Gew.-% P₄O₁₀) vorgelegt. Der auf 90°C beheizte Dosiertrichter wird mit einer Lösung von 2,2 g 4-(2-Nitrophenyl)-n-buttersäurechlorid (NPBSC, 9,67 mmol) in 20 g Dibenzyltoluol (Isomerengemisch, Handelsname Marlotherm SH, Sasol Germany GmbH) beschickt. Die Apparatur wird evakuiert und unter Rühren auf Rückfluss erhitzt (166°C, 0,1 mbar). Anschließend tropft man die Lösung von NPBSC kontinuierlich innerhalb von 20 Minuten zu der vorgelegten Lösung von Polyphosphorsäure und Dibenzyltoluol (Isomerengemisch, Handelsname Marlotherm SH, Sasol Germany GmbH) zu. Unmittelbar nach dem Beginn des Zutropfens der Säurechloridlösung steigt der Druck in der Apparatur durch Bildung von gasförmiger Salzsäure auf ca. 2 mbar. Die Destillatabnahme wird entsprechend dem Zulauf eingestellt, so dass das Reaktionsvolumen im Sumpfkolben konstant bleibt. Nach vollständiger Zugabe der Lösung wird mit 20 g Dibenzyltoluol (Isomerengemisch, Handelsname Marlotherm SH, Sasol Germany GmbH) nachgespült und sauber destilliert.

Das Destillat enthält 783 mg 5-Nitro-3,4-dihydro-1(2H)-naphthalinon (NT) und 642 mg NPBSC (ermittelt durch GC/ Interne Standard-Methode):

| | |
|---|---|
| Ausbeute NT | 42,7 % |
| Umsatz NPBSC | 71,0 % |
| Selektivität | 62,8 % |

## Patentansprüche

1. Verfahren zur Herstellung von 5-Nitro-3,4-dihydro-1(2H)-naphthalinon, das einen Schritt enthält, in dem man 4-(2 Nitrophenyl)-n-butyronitril zu 4-(2-Nitrophenyl)-n-buttersäure umsetzt.

2. Verfahren nach Anspruch 1, enthaltend die Schritte
a) Umsetzung von 4-(2-Nitrophenyl)-n-butyronitril mit einer Säure oder einer Base zu 4-(2-Nitrophenyl)-n-buttersäure,
b) Cyclisierung der in Schritt a) gebildeten 4-(2-Nitrophenyl)-n-buttersäure zu 5-Nitro-3,4-dihydro-1(2H)-naphthalinon

3. Verfahren nach Anspruch 1, enthaltend die Schritte
a) Umsetzung von 4-2-Nitrophenyl)-n-butylronitril mit einer Säure oder einer Base zu 4-(2-Nitropheyl)-n-buttersäure,
b) Chlorierung der in Schritt a) gebildeten 4-(2-Nitrophenyl)-n-butter-säure zum 4-(2-Nitrophenyl)-n-buttersäurechlorid,
c) Cyclisieren des in Schritt b) gebildeten 4-(2-Nitrophenyl)-n-buttersäu-rechlorids zu 5-Nitro-3,4-dihydro-1(2H)-naphthalinon

4. Verfahren nach Anspruch 1, wobei man das 5-Nitro-3,4-dihydro-1(2(H)-Naphthalinon weiter zu 1,5-Naphthyldiamin umsetzt.

5. Verfahren nach einem der Ansprüche 1 bis 3, bei dem man anschließend
a) das 5-Nitro-3,4-dihydro(2H)-naphthalinon zu 5-Nitro-3,4-dihydro-1-naphthylamin bzw. dem tautomeren 5-Nitro-3,4-dihydro-1(2H)-naphthylimin aminiert, und anschließend
b) das in Schritt a) gebildete 5-Nitro-3,4-dihydro-1-naphthylamin bzw. das tautomere 5-Nitro-3,4-dihydro-1(2H)-naphthylimin zu -5-Nitro-1-naphthylamin und/oder 5-Nitroso-1-naphthylamin aromatisiert, und anschließend
c) das in Schritt b) gebildete 5-Nitro-1-naphthylamin und / oder 5-Nitroso-1-naphthylamin zu 1,5-Naphalindiamin hydriert.

6. Verfahren nach einem der Ansprüche 4 oder 5, wobei βend das 1,5 -Naphthalindiamin phosgeniert wird zu 1,5 -Naphthalindiisocyanat.

## Claims

1. Process for producing 5-nitro-3,4-dihydro-1(2H)-naphthalenone, comprising a step in which 4-(2-nitrophenyl)-n-butyronitrile is reacted to produce 4-(2-nitrophenyl)-n-butyric acid.

2. Process according to Claim 1, comprising the steps of
a) reacting 4-(2-nitrophenyl)-n-butyronitrile with an acid or a base to produce 4-(2-nitrophenyl)-n-butyric acid,
b) cyclizing the 4-(2-nitrophenyl)-n-butyric acid formed in step a) to produce 5-nitro-3,4-dihydro-1(2H)-naphthalenone.

3. Process according to Claim 1, comprising the steps of
a) reacting 4-(2-nitrophenyl)-n-butyronitrile with an acid or a base to produce 4-(2-nitrophenyl)-n-butyric acid,
b) chlorinating the 4-(2-nitrophenyl)-n-butyric acid formed in step a) to produce 4-(2-nitrophenyl)-n-butyryl chloride,
c) cyclizing the 4-(2-nitrophenyl)-n-butyryl chloride formed in step b) to produce 5-nitro-3,4-dihydro-1(2H)-naphthalenone.

4. Process according to Claim 1, where the 5-nitro-3,4-dihydro-1(2H)-naphthalenone is reacted further to produce 1,5-naphthyldiamine.

5. Process according to any of Claims 1 to 3, in which subsequently
a) the 5-nitro-3,4-dihydro-1(2H)-naphthalenone is aminated to produce 5-nitro-3,4-dihydro-1-naphthylamine and/or the tautomeric 5-nitro-3,4-dihydro-1(2H)-naphthylimine, and subsequently
b) the 5-nitro-3,4-dihydro-1-naphthylamine and/or the tautomeric 5-nitro-3,4-dihydro-1(2H)-naphthylimine formed in step a) are/is aromatized to produce 5-nitro-1-naphthylamine and/or 5-nitroso-1-naphthylamine, and subsequently
c) the 5-nitro-1-naphthylamine and/or 5-nitroso-1-naphthylamine formed in step b) are/is hydrogenated to produce 1,5-naphthalenediamine.

6. Process according to either of Claims 4 and 5, where subsequently the 1,5-naphthalenediamine is phosgenated to produce 1,5-naphthalene diisocyanate.

## Revendications

1. Procédé pour la préparation de 5-nitro-3,4-dihydro-1(2H)-naphtalénone, qui comporte une étape dans laquelle on convertit le 4-(2-nitrophényl)-n-butyronitrile en acide 4-(2-nitrophényl)-n-butyrique.

2. Procédé selon la revendication 1, comportant les étapes suivantes:
a) conversion de 4-(2-nitrophényl)-n-butyronitrile avec un acide ou une base en acide 4-(2-nitrophényl)-n-butyrique,
b) cyclisation de l'acide 4-(2-nitrophényl)-n-butyrique obtenu à l'étape a) en 5-nitro-3,4-dihydro-1(2H)-naphtalénone.

3. Procédé selon la revendication 1, comportant les étapes suivantes:
a) conversion de 4-(2-nitrophényl)-n-butyronitrile avec un acide ou une base en acide 4-(2-nitrophényl)-n-butyrique,
b) chloration de l'acide 4-(2-nitrophényl)-n-butyrique obtenu à l'étape a) en chlorure de l'acide 4-(2-nitrophényl)-n-butyrique,
c) cyclisation du chlorure de l'acide 4-(2-nitrophényl)-n-butyrique obtenu à l'étape b) en 5-nitro-3,4-dihydro-1(2H)-naphtalënone.

4. Procédé selon la revendication 1, dans lequel on convertit la 5-nitro-3,4-dihydro-1(2H)-naphtalénone en 1,5-naphtyldiamine.

5. Procédé selon l'une quelconque des revendications 1 à 3, dans lequel
a) on amine ensuite la 5-nitro-3,4-dihydro-1(2H)-naphtalénone en 5-nitro-3,4-dihydro-1-naphtylamine ou en 5-nitro-3,4-dihydro-1(2H)-naphtylimine, et ensuite
b) on aromatise la 5-nitro-3,4-dihydro-1-naphtylamine ou la tautomère 5-nitro-3,4-dihydro-1(2H)-naphtylimine tautomère formée à l'étape a) en 5-nitro-1-naphtylamine et/ou en 5-nitroso-1-naphtylamine, et ensuite
c) on hydrate la 5-nitro-1-naphtylamine et/ou la 5-nitroso-1-naphtylamine formée à l'étape b) en 1,5-naphtalènediamine.

6. Procédé selon l'une quelconque des revendications 4 ou 5, dans lequel on effectue ensuite la phosgénisation de la 1,5-naphtalènediamine en 1,5-naphtalènediisocyanate.
